(19) Europäisches Patentamt
European Patent Office
Office européen des brevets

(11) Veröffentlichungsnummer: **0 348 764 B1**

(12) **EUROPÄISCHE PATENTSCHRIFT**

(45) Veröffentlichungstag der Patentschrift: **01.06.94**

(51) Int. Cl.⁵: **C07C 69/18**, C07C 67/293

(21) Anmeldenummer: **89111037.1**

(22) Anmeldetag: **19.06.89**

# (54) Alkantriolmonoester und ein Verfahren zu ihrer Herstellung.

(30) Priorität: **25.06.88 DE 3821479**

(43) Veröffentlichungstag der Anmeldung:
**03.01.90 Patentblatt 90/01**

(45) Bekanntmachung des Hinweises auf die Patenterteilung:
**01.06.94 Patentblatt 94/22**

(84) Benannte Vertragsstaaten:
**BE CH DE FR GB IT LI**

(56) Entgegenhaltungen:
**US-A- 4 337 271**

**TETRAHEDRON, Band 40, 1984, Seiten 3471-3479, Oxford, GB; K. MORI et al.:"Synthesis of all of the four energetically possible stereoisomers of 7-ethyl-2-methyl-1,6-dioxaspiro[4,5]decane"**

(73) Patentinhaber: **BASF Aktiengesellschaft**
**Carl-Bosch-Strasse 38**
**D-67063 Ludwigshafen(DE)**

(72) Erfinder: **Fischer, Rolf, Dr.**
**Bergstrasse 98**
**D-6900 Heidelberg(DE)**

EP 0 348 764 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Diese Erfindung betrifft Alkantriolmonoester und ein Verfahren zu ihrer Herstellung durch Umsetzung von α,ω-Diolen mit Estern von Alkenolen in Gegenwart von Radikalbildnern.

Es ist bekannt, daß sich primäre und sekundäre Alkanole in Gegenwart von Radikalbildnern, wie tert.-Butylhydroperoxid oder Di-tert.-butylperoxid, mit 1-Alkenen zu höheren Alkoholen (J. Amer. Chem. Soc. 76, 450 (1953)) und mit Acrylsäureestern zu 5-Alkylbutyrolactonen (Izv. Akad. SSSR, 1964, 894) umsetzen lassen. Über eine Umsetzung von Diolen mit Olefinen in Gegenwart von Radikalbildnern ist lediglich bekannt, daß man aus α,ω-Diolen, wie 1,4-Butandiol, in Gegenwart von Di-tert.-butylperoxid mit Acrylsäureestern (5-(ω-Hydroxyalkyl)butyrolactone erhält (DE-OS 28 18 126). Eine Umsetzung von α,ω-Diolen mit 1-Alkenylacetaten wurde dagegen bisher nicht beschrieben.

Es wurde nun gefunden, daß man Alkantriolmonoester der Formel

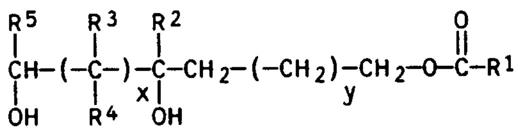

I,

in der x und y ganze Zahlen von 0 bis 6, $R^1$ ein Wasserstoffatom, einen Alkylrest mit 1 bis 12 C-Atomen, einen Cycloalkylrest, einen Aralkylrest oder einen Arylrest und die Reste $R^2$, $R^3$, $R^4$ und $R^5$ jeweils ein Wasserstoffatom oder einen Alkylrest mit 1 bis 4 C-Atomen bedeuten, erhält, wenn man α,ω-Diole der allgemeinen Formel

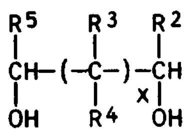

II,

in der x und die Reste $R^2$ bis $R^5$ die obengenannte Bedeutung haben, mit Alkenolestern der allgemeinen Formel

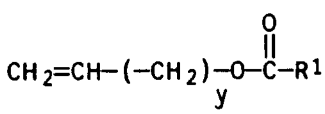

III,

in der y und $R^1$ die obengenannte Bedeutung haben, in Gegenwart von Radikalbildnern bei höheren Temperaturen umsetzt.

Wie die negativen Ergebnisse der Vergleichsbeispiele 11 und 12 zeigen, in denen anstelle von Allylacetat und Butenylacetat Allylalkohol und 1-Buten-4-ol verwendet wurden, war der erfolgreiche Verlauf der erfindungsgemäßen Reaktion nicht vorauszusehen.

Die Erfindung betrifft weiterhin die neuen Alkantriolmonoester der allgemeinen Formel

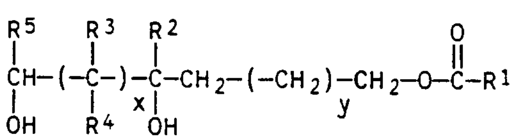

I,

in der x ganze Zahlen von 1 bis 6, y ganze Zahlen von 0 bis 6, $R^1$ ein Wasserstoffatom, einen Alkylrest mit 1 bis 12 C-Atomen, einen Cycloalkylrest, einen Aralkylrest oder einen Arylrest und die Reste $R^2$, $R^3$, $R^4$ und $R^5$ jeweils ein Wasserstoffatom oder einen Alkylrest mit 1 bis 4 C-Atomen bedeuten.

In den neuen Alkantriolmonoestern der Formel I bedeuten x eine ganze Zahl von 1 bis 6, y eine ganze Zahl von O bis 6, vorzugsweise 0 bis 4, $R^1$ ein Wasserstoffatom, einen Alkylrest mit 1 bis 12, vorzugsweise 1 bis 6 C-Atomen, einen Cycloalkylrest, wie einen Cyclopentyl- oder Cyclohexylrest, einen Aralkylrest, wie

einen Benzylrest oder einen Arylrest, wie den Phenylrest. Die Reste $R^2$ bis $R^5$ können Alkylreste mit 1 bis 4 C-Atomen, wie Methyl, Ethyl, Propyl oder Butyl sein; bevorzugt stehen sie für Wasserstoffatome.

Die neuen Alkantriolmonoester der Formel I sind vielseitig abwandelbare Zwischenprodukte, die sich z.B. zu den entsprechenden Triolen verseifen oder umestern lassen.

Die Reaktion zur Herstellung der Alkantriolmonoester läßt sich z.B. für den Fall der Umsetzung von 1,4-Butandiol mit Allylacetat und Di-tert.-butylperoxid als Radikalbildner durch die folgende Formelgleichung wiedergeben:

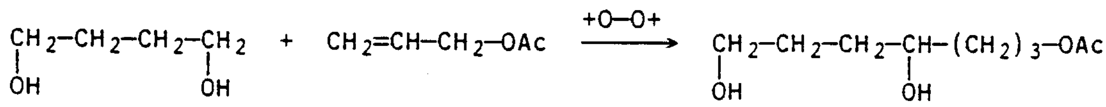


Als α,ω-Diole der Formel II seien z.B. Ethylenglykol, 1,4-Butandiol, 1,3-Propandiol, 1,5-Pentandiol, 1,6-Hexandiol, 1,7-Heptandiol und 1,8-Octandiol genannt.

Alkenolester der Formel III sind z.B. Vinylacetat, Allylacetat, 1-Butenyl-4-ol-acetat, Allylpropionat, Vinylpropionat, 1-Pentenyl-5-ol-formiat und 1-Hexenyl-6-ol-acetat.

Die erfindungsgemäße Umsetzung wird bei höheren Temperaturen durchgeführt. Beispielsweise wählt man Temperaturen von 100 bis 200 °C, insbesondere 140 bis 170 °C. Im allgemeinen arbeitet man bei Normaldruck; es kann aber auch empfehlenswert sein, die Umsetzung bei erhöhtem Druck, z.B. bis zu 20 bar, durchzuführen.

Um die Polymerisation der Alkenolester III zurückzudrängen, ist es vorteilhaft, die Diole der Formel II im Überschuß einzusetzen. So wählt man z.B. ein Molverhältnis der Verbindung II zu III von 3:1 bis 40:1, vorzugsweise von 5:1 bis 15:1. Durch Arbeiten unter Inertgas läßt sich die Ausbeute an den erwünschten Verbindungen der Formel I oftmals steigern. Hierdurch werden durch den Luftsauerstoff bewirkte Nebenreaktionen unterbunden.

Als Radikalbildner sind z.B. Sauerstoff und alle Peroxide und Azoverbindungen geeignet, die üblicherweise auch bei der Polymerisation von olefinischen Monomeren verwendet werden. Beispiele hierfür sind Azoverbindungen, wie Azoisobutyronitril, Hydroperoxide, wie tert.-Butylhydroperoxid und Cyclohexylhydroperoxid, Diacylperoxide, wie Dibenzoylperoxid, Peroxysäuren und entsprechende Ester, wie m-Chlorperbenzoesäure, Perbenzoesäure, tert.-Butylperbenzoat und Dialkylperoxide, wie Dicyclohexylperoxid, tert.-Butylcyclohexylperoxid und insbesondere Di-tert.-butylperoxid. Im allgemeinen verwendet man 0,05 bis 0,3 Mol, insbesondere 0,1 bis 0,2 Mol des Radikalbildners, bezogen auf den jeweiligen Alkenolester.

In den meisten Fällen ist es nicht notwendig, ein Lösungsmittel zuzusetzen, da das im Überschuß eingesetzte Diol gleichzeitig als Lösungsmittel dient. Falls ein zusätzliches Lösungsmittel erforderlich ist, so verwendet man ein gegenüber den Radikalbildnern, aber auch gegenüber den Verbindungen der Formeln I bis III inertes Lösungsmittel. Lösungsmittel dieser Art sind z.B. aromatische Kohlenwasserstoffe, wie Chlorbenzole.

Die Reaktionszeiten liegen im allgemeinen bei 1 bis 4 Stunden. Es empfiehlt sich, durch geeignete Analysenmethoden festzustellen, ob Reaktionszeit und Reaktionstemperatur ausreichen, um den Radikalbildner vollständig abzubauen. Sollte ein solcher Abbau nicht eingetreten sein, so wird der Radikalbildner durch geeignete Maßnahmen, z.B. durch Zugabe von Reduktionsmittel, nachträglich zerstört.

Die Aufarbeitung der Alkantriolmonoester kann meist durch Destillation erfolgen. Hierzu wird zunächst das überschüssige α,ω-Diol abdestilliert. Der so erhaltene Rückstand kann entweder durch Destillation oder Kristallisation zu reinem Alkantriolmonoester aufgearbeitet werden.

Beispiel 1

Herstellung von 1,4-Dihydroxy-7-acetoxyheptan (siehe Formel I mit x = 2, y = 1, R = $CH_3$)

In einem mit Rührer, 2 Tropftrichtern und Innenthermometer ausgerüsteten 2-1-Vierhalskolben wurden 810 g 1,4-Butandiol (9 mol) unter Stickstoff auf 160 °C erhitzt. Bei 160±2 °C wurden innerhalb von 75 Minuten gleichzeitig 90 g frisch destilliertes Allylacetat (0,9 mol) und 26 g Di-tert.-butylperoxid (0,18 mol) zugegeben. Nach beendeter Zugabe wurde das Reaktionsgemisch noch eine Stunde bei 160 °C nachgerührt.

Nach dem Abkühlen wurden 720 g unumgesetztes 1,4-Butandiol (8 mol) über eine Claisenbrücke abdestilliert. Durch fraktionierende Destillation des Rückstands wurden 87 g 1,4-Dihydroxy-7-acetoxyheptan vom Siedepunkt 140 bis 148 °C/0,1 mbar (51 %, bezogen auf eingesetztes Allylacetat) erhalten.

Beispiele 2 bis 10

Bei den in Beispiel 1 angegebenen Temperaturen und Molverhältnissen wurden die aus der Tabelle ersichtlichen Reaktionspartner II, III und Di-tert.-butylperoxid entsprechend umgesetzt. Die angegebenen Ausbeuten der Alkantriolmonoester wurden nach Destillation bestimmt; sie beziehen sich auf den eingesetzten Alkenolester der Formel III

| Beispiel | eingesetzte Verbindungen II und III | | | Ausbeute an I (% d. Th.) |
|---|---|---|---|---|
| | x | y | $R^1$ | |
| 2 | 0 | | | - |
| 3 | 1 | | | 37 |
| 4 | 2 | 0 | | 43 |
| 5 | 3 | | | 51 |
| 6 | 4 | | $CH_3$ | 45 |
| 7 | 1 | | | 31 |
| 8 | 3 | 1 | | 58 |
| 9 | 4 | | | 51 |
| 10 | 2 | 2 | | 48 |

Beispiel 11 (Vergleich)

Umsetzung von 1,4-Butandiol mit Allylalkohol

Beispiel 1 wurde wiederholt, wobei jedoch anstelle von Allylacetat Allylalkohol verwendet wurde. Es wurden nur Spuren an 1.4.7-Heptantriol gaschromatographisch gefunden.

Beispiel 12 (Vergleich)

Umsetzung von 1,4-Butandiol mit 1-Buten-4-ol

Beispiel 10 wurde wiederholt, wobei jedoch anstelle von 1-Buten-4-ol-acetat 1-Buten-4-ol verwendet wurde. Es wurden nur geringe Mengen an 1.4.8-Octantriol gaschromatographisch ermittelt.

## Patentansprüche

**1.** Verfahren zur Herstellung von Alkantriolmonoestern der allgemeinen Formel

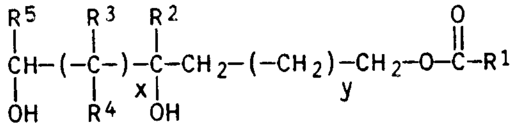

I,

in der x und y für ganze Zahlen von 0 bis 6 stehen, $R^1$ ein Wasserstoffatom, einen Alkylrest mit l bis 12 C-Atomen, einen Cycloalkylrest, einen Aralkylrest oder einen Arylrest und die Reste $R^2$, $R^3$, $R^4$ und $R^5$ jeweils ein Wasserstoffatom oder einen Alkylrest mit 1 bis 4 C-Atomen bedeuten, dadurch gekennzeichnet, daß man $\alpha,\omega$-Diole der allgemeinen Formel

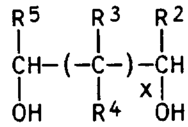

II,

in der x und die Reste $R^2$ bis $R^5$ die obengenannte Bedeutung haben, mit Alkenolestern der allgemeinen Formel

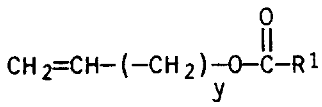

III,

in der y und $R^1$ die obengenannte Bedeutung haben, in Gegenwart von Radikalbildnern bei höheren Temperaturen umsetzt.

**2.** Verfahren nach Anspruch 1, dadurch gekennzeichnet, daß man die Umsetzung bei Temperaturen von 100 bis 200 °C vornimmt.

**3.** Verfahren nach Anspruch 1, dadurch gekennzeichnet, daß man ein Molverhältnis zwischen dem $\alpha,\omega$-Diol und dem Alkenolester von 3:1 bis 40:1 einhält.

**4.** Verfahren nach Anspruch 1, dadurch gekennzeichnet, daß man pro Mol Alkenolester 0,05 bis 0,3 Mol des Radikalbildners anwendet.

**5.** Alkantriolmonoester der allgemeinen Formel

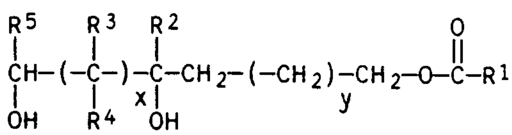

I,

in der x ganze Zahlen von 1 bis 6, y ganze Zahlen von 0 bis 6, $R^1$ ein Wasserstoffatom, einen Alkylrest mit 1 bis 12 C-Atomen, einen Cycloalkylrest, einen Aralkylrest oder einen Arylrest und die Reste $R^2$, $R^3$, $R^4$ und $R^5$ jeweils ein Wasserstoffatom oder einen Alkylrest mit 1 bis 4 C-Atomen bedeuten.

## Claims

**1.** A process for preparing alkanetriol monoesters of the general formula

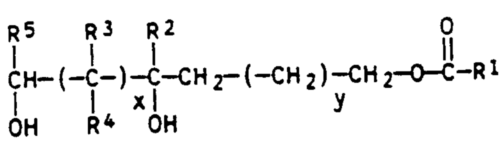

I,

where x and y are integers from 0 to 6, $R^1$ is a hydrogen atom, an alkyl radical having 1 to 12 C atoms, a cycloalkyl radical, an aralkyl radical or an aryl radical, and the radicals $R^2$, $R^3$, $R^4$ and $R^5$ in each case are a hydrogen atom or an alkyl radical having 1 to 4 C atoms, which comprises reacting $\alpha,\omega$-diols of the general formula

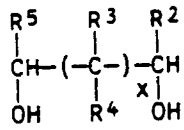 II,

where x and the radicals $R^2$ to $R^5$ have the abovementioned meanings, with alkenol esters of the general formula

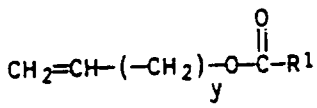 III,

where y and $R^1$ have the abovementioned meanings, at relatively high temperatures in the presence of free radical initiators.

**2.** A process as claimed in claim 1, wherein the reaction is carried out at from 100 to 200 °C.

**3.** A process as claimed in claim 1, wherein a molar ratio between the α,ω-diol and the alkenol ester of 3:1-40:1 is maintained.

**4.** A process as claimed in claim 1, wherein 0.05-0.3 mol of the free radical initiator is used per mole of alkenol ester.

**5.** An alkanetriol monoester of the general formula

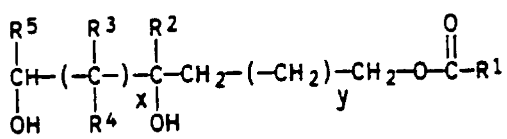 I,

where x is integers from 1 to 6, y is integers from 0 to 6, $R^1$ is a hydrogen atom, an alkyl radical having 1 to 12 C atoms, a cyclolalkyl radical, an aralkyl radical or an aryl radical, and the radicals $R^2$, $R^3$, $R^4$ and $R^5$ in each case are a hydrogen atom or an alkyl radical having 1 to 4 C atoms.

## Revendications

**1.** Procédé de préparation de monoesters d'alcanetriol de formule générale

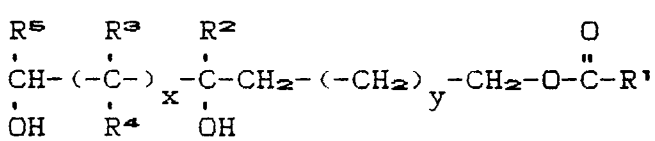 I

dans laquelle x et y sont mis pour des nombres entiers de 0 a 6, $R^1$ représente un atome d'hydrogène, un reste alkyle à 1-12 atomes de carbone, un reste cycloalkyle, un reste aralkyle ou un reste aryle, et les restes $R^2$, $R^3$, $R^4$ et $R^5$ représentent chacun un atome d'hydrogène ou un reste alkyle à 1-4 atomes de carbone, caractérisé en ce qu'on fait réagir des α,ω-diols de formule générale

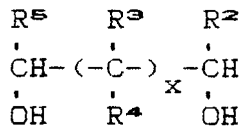 II

dans laquelle x et les restes $R^2$ a $R^6$ ont les significations données ci-dessus, avec des esters d'alcénol de formule générale

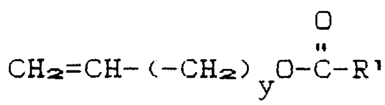 III

dans laquelle y et $R^1$ ont les significations données ci-dessus, en présence d'amorceurs radicalaires a température élevée.

**2.** Procédé selon la revendication 1, caractérisé en ce qu'on conduit la réaction a des températures de 100 à 200 ° C.

**3.** Procédé selon la revendication 1, caractérisé en ce qu'on maintient un rapport molaire de 3:1 à 40:1 entre l'α,ω-diol et l'ester d'alcénol.

**4.** Procédé selon la revendication 1, caractérisé en ce qu'on utilise, par mole d'ester d'alcénol, de 0,05 à 0,3 mole de l'amorceur radicalaire.

**5.** Monoesters d'alcanetriol de formule générale

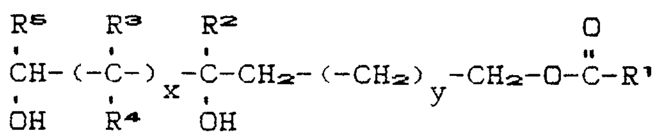 I

dans laquelle x est un nombre entier de 1 à 6, y est un nombre entier de 0 a 6, $R^1$ représente un atome d'hydrogène, un reste alkyle a 1-12 atomes de carbone, un reste cycloalkyle, un reste aralkyle ou un reste aryle, et les restes $R^2$, $R^3$, $R^4$ et $R^5$ représentent chacun un atome d'hydrogène ou un reste alkyle à 1-4 atomes de carbone.